# EUROPÄISCHE PATENTSCHRIFT

(11) **EP 0 565 058 B1**
(45) Veröffentlichungstag und Bekanntmachung des Hinweises auf die Patenterteilung: **23.10.1996**
(21) Anmeldenummer: 93105694.9
(22) Anmeldetag: 06.04.1993
(51) Int. Cl.: A61F 13/15

(54) **Wegwerfwindel**
Disposable diaper
Couche à jeter

(30) Priorität: 06.04.1992 JP 84050/92
(43) Veröffentlichungstag der Anmeldung: 13.10.1993
(73) Patentinhaber: UNI-CHARM CORPORATION, Kawanoe-shi Ehime-ken (JP)
(72) Erfinder: Kitaoka, Hideaki, Funabashi-shi, Chiba-ken (JP)
(74) Vertreter: Sperling, Rüdiger, Dipl.-Ing.

(56) Entgegenhaltungen:
- EP-A- 0 359 410
- EP-A- 0 376 022
- EP-A- 0 459 178
- EP-A- 0 486 006

## Beschreibung

Die Erfindung betrifft eine Wegwerfwindel, die zum Absorbieren und Aufnehmen von Ausscheidungen des menschlichen Körpers verwendet wird.

Die japanische Gebrauchsmusteranmeldung Amtsblatt-Nr. 1974-120439 zeigt eine Windelabdeckung auf, die eine Decklage aufweist, in deren Mittelzone eine Öffnung ausgebildet ist, die sich in Längsrichtung der Decklage weiter erstreckt als in Querrichtung derselben, wobei die Öffnung entlang ihrem Umfangsrand mit einem in Längsrichtung dehnbaren elastischen Element versehen ist, um so eine elastische Linie in Form eines geschlossenen Kreises zu bilden. Die japanische Patentanmeldung Amtsblatt-Nr. 1986-41304 zeigen ebenfalls eine Wegwerfwindel mit einer Decklage auf, in deren Mittelbereich eine Öffnung ausgebildet ist, die sich in Längsrichtung weiter als in Querrichtung der Decklage erstreckt, wobei die Öffnung entlang ihren seitlich gegenüberliegenden Seitenrändern jeweils mit elastischen Elementen versehen ist.

Bei dieser Windelabdeckung und Windel, die beide mit Öffnungen versehen sind, fließen die Ausscheidungen durch die Öffnung in eine zwischen der Decklage und einer weiteren Decklage unterhalb der ersten Decklage gebildeten Tasche und werden darin zurückgehalten. Eine ähnliche Windel ist aus des EP-A-0 486 006 bekannt.

Der Benutzer dieser Windeln trägt jedoch dieselben nicht immer in der Weise, daß die Öffnung ordnungsgemäß ausgerichtet ist, so daß sie die Harnorgane und/oder den Anus bedeckt, mit anderen Worten kann diese Öffnung manchmal aus ihrer ordnungsgemäßen Position bezüglich den Harnorganen und/oder dem Anus geraten, mit dem Ergebnis, daß nicht alle Ausscheidungen durch diese Öffnung fließen und ein Teil der Ausscheidungen sich über die oberste Lage verteilt. Somit ist der durch das Vorsehen der obersten Lage zusätzlich zur Decklage und das Ausbilden der Öffnung in dieser obersten Lage erwartete Effekt im wesentlichen verloren und die sich über die oberste Lage verteilenden Ausscheidungen kommen in Berührung mit der Haut des Benutzers, wo sie Hautkrankheiten verursachen und zumindest dem Benutzer ein unangenehmes Gefühl vermitteln.

Es ist eine Aufgabe der Erfindung, eine Wegwerfwindel aufzuzeigen, die so verbessert ist, daß die gesamten Ausscheidungen zuverlässig durch die Öffnung fließen.

### BESCHREIBUNG DER ERFINDUNG

Die vorstehend dargelegte Aufgabe wird gemäß der Erfindung durch eine Wegwerfwindel gelöst, die einen integralen Schichtkörper umfaßt, der sich aus einer flüssigkeitsdurchlässigen ersten Decklage, einer flüssigkeitsundurchlässigen Außenlage, einem flüssigkeitsabsorbierenden Kern, der zwischen die erste Decklage und die Außenlage gelegt ist, und einer flüssigkeitsbeständigen zweiten Decklage zusammensetzt, die über die erste Decklage gelegt ist, wobei die zweite Decklage im wesentlichen in ihrem Mittelbereich eine Öffnung aufweist, die sich in Längsrichtung der zweiten Decklage weiter als in Querrichtung derselben erstreckt, wobei die zweite Decklage entlang ihrem äußeren Umfang mit der ersten Decklage verbunden ist und wobei in Längsrichtung elastische Elemente entlang dem Umfangsrand der Öffnung an der zweiten Decklage angebracht sind, dadurch gekennzeichnet, daß zwei flüssigkeitsbeständige Klappen von den in Querrichtung gegenüberliegenden Seitenrändern der Öffnung ausgehen, die wenigstens entlang ihren äußeren Seitenrändern jeweils mit elastischen Elementen versehen sind und die in Längsrichtung gegenüberliegenden Enden der jeweiligen Klappen mit der zweiten Decklage verbunden sind.

Vorzugsweise ist die zweite Decklage in ihrer Mitte mit einer in Längsrichtung verlaufenden Schnittlinie, die sich über eine gewünschte Länge erstreckt, versehen, und die zweite Decklage ist weiter an in Längsrichtung gegenüberliegenden Enden der in Längsrichtung verlaufenden Schnittlinie mit querverlaufenden Schnittlinien jeweils versehen, so daß Abschnitte der zweiten Decklage, die durch diese Schnittlinien begrenzt sind, nach außen gefaltet werden können, um die jeweiligen Klappen zu bilden.

Vorzugsweise ist zwischen den Unterflächen der jeweiligen Klappen und der Oberfläche der zweiten Decklage ein weiteres Paar elastischer Elemente vorgesehen, die sich entlang den jeweiligen Faltlinien der Klappen erstrecken.

Bei der vorstehend beschriebenem Aufbau entsprechenden erfindungsgemäßen Windel erheben sich die Klappen nach oben und außen von ihren inneren Seitenrändern unter der Kontraktion der elastischen Elemente, die entlang ihren äußeren Seitenrändern vorgesehen sind, um so schräg verlaufende Seitenwände zu bilden, die einander gegenüberliegend genau wie die Wände eines Einfülltrichters angeordnet sind, die einander gegenüberliegen. Ausscheidungen werden durch diese geneigten Seitenwände in die Öffnung geführt. Der flüssige Anteil der Ausscheidungen wird durch die erste Decklage im Kern absorbiert, während der feste Anteil der Ausscheidungen in eine Tasche eingeführt wird, die zwischen der ersten Decklage und der zweiten Decklage gebildet ist.

### KURZE BESCHREIBUNG DER ZEICHNUNG

Die Erfindung wird nachfolgend anhand eines Beispiels unter Bezug auf die beiliegenden Figuren näher erläutert, wobei:
- Fig. 1: eine perspektivische Darstellung der Innenseite einer Wegwerfwindel zeigt, die als eine Ausführungsform der Erfindung aufgebaut ist;
- Fig. 2: eine Schnittdarstellung dieser Ausführungsform in vergrößertem Maßstab entlang einer Linie 2-2 in Fig. 1 zeigt;
- Fig. 3: eine Draufsicht zur Darstellung von Öffnungen einer zweiten Decklage wie auch des ersten Schrittes in einem Verfahren zum Anbringen von elastischen Elementen an die jeweiligen Öffnungen zeigt; und
- Fig. 4: eine Draufsicht zur Darstellung der Öffnungen der zweiten Decklage wie auch des zweiten Schrittes im Verfahren zum Anbringen der elastischen Elemente an den jeweiligen Öffnungen darstellt.

### BEVORZUGTE AUSFÜHRUNGSFORMEN DER ERFINDUNG

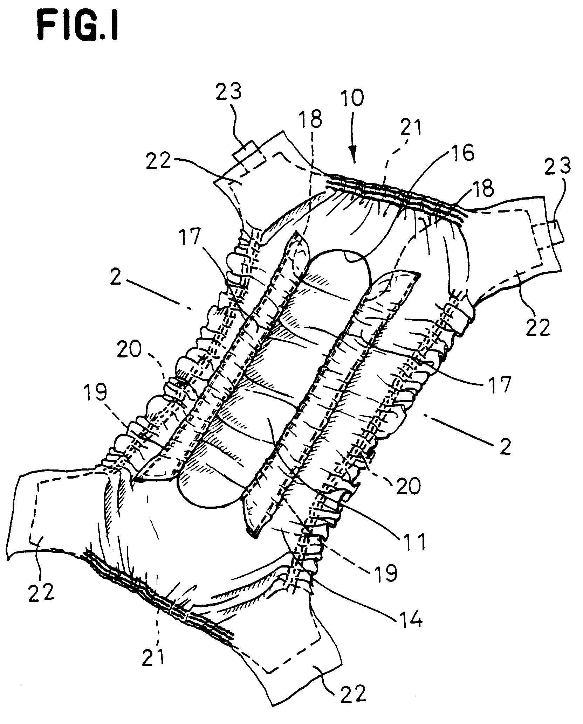

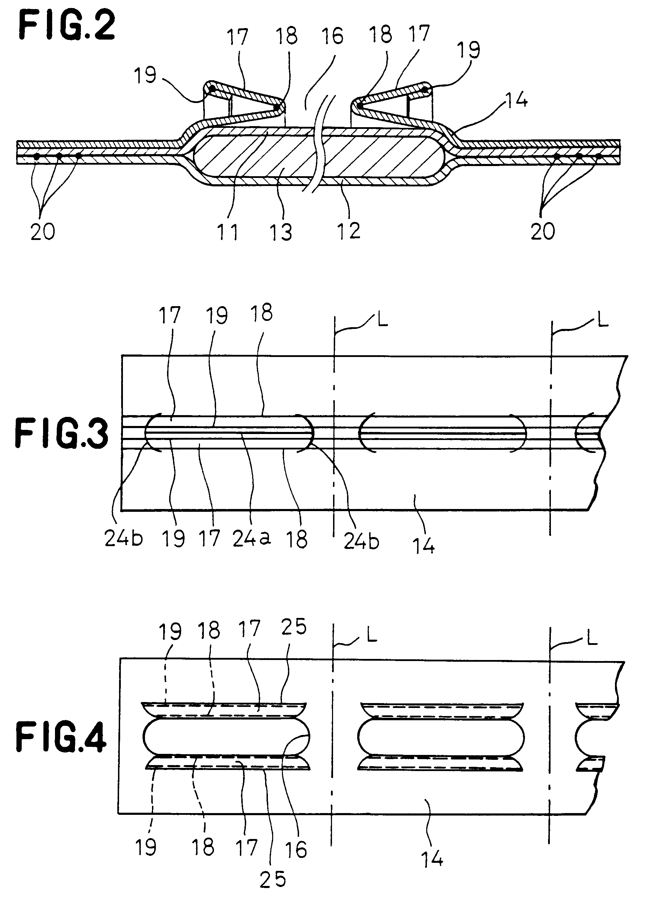

Wie in Fig. 1 und 2 gezeigt, umfaßt eine Windel 10 einen integralen Schichtkörper 15, der sich aus einer flüssigkeitsdurchlässigen ersten Decklage 11, einer flüssigkeitsundurchlässigen Außenlage 12, einem flüssigkeitsabsorbierenden Kern 13, der zwischen diese gelegt ist, und einer flüssigkeitsbeständigen zweiten Decklage 14 zusammensetzt. Die zweite Decklage 14 ist im Mittelbereich mit einer Öffnung 16 versehen, die in Längsrichtung länger als in Querrichtung der Lage 14 ist und in Längsrichtung gegenüberliegende Enden aufweist, die jeweils Kreisbögen beschreiben. Die Öffnung 16 kann zumindest innerhalb des Schrittbereiches ausgebildet sein.

Zwischen den seitlich gegenüberliegenden Rändern der ersten Decklage 11 und den seitlich gegenüberliegenden Rändern der Außenlage 12, die beiderseits des flüssigkeitsabsorbierenden Kernes 13 sich nach außen erstrecken, sind mehrere elastische Elemente 20, die jeweils wiederum eine Anzahl elastischer Gummifäden umfassen, in gestrecktem Zustand unter Verwendung von Heißschmelzkleber (nicht dargestellt) jeweils angebracht, so daß sie in Längsrichtung der Lagen dehnbar sind und dicht um die Beine des Benutzers passen. In ähnlicher Weise sind zwischen den in Längsrichtung gegenüberliegenden Enden der ersten Decklage 11 und den zugehörigen Enden der Decklage 12 mehrere elastische Elemente 21, die jeweils wiederum mehrere elastische Gummifäden umfassen, vorgesehen, so daß sie in Querrichtung der Lagen dehnbar sind und dicht um die Hüften des Benutzers passen.

Die erste Decklage 11 kann aus Fliesstoff, poröser Plastikfolie oder ähnlichem hergestellt sein, die Außenlage 12 kann aus Kunststoffolie, laminierten Lagen dieser Kunststoffolie und Fliesstoff oder ähnlichem hergestellt sind, der flüssigkeitsabsorbierende Kern 13 kann aus einer Mischung von Faserpulpe und hochabsorbierendem Polymerpulver oder ähnlichem hergestellt sein. Die zweite Decklage 14 und Klappen 17 sind vorzugsweise aus wasserabstoßendem und hochluftdurchlässigem Fliesstoff hergestellt. Es sei angemerkt, daß in der Beschreibung der Erfindung sich "füssigkeitsbeständiges" Material auf Material bezieht, daß einen ausreichenden Grad von Wasserabstoßfähigkeit aufweist, um das leichte Durchdringen von flüssigen Ausscheidungen durch dieses zu verhindern, wenn die Windel an den Körper des Benutzers angelegt ist.

Wie in Fig. 1 gezeigt, hat die Windel 10 zwei Paar flügelähnliche Laschen 22, die sich von den seitlich gegenüberliegenden Seiten der Hüftlinie jeweils nach außen erstrecken, und die freien Enden von Bandbefestigungen 23, die an den jeweiligen rückwärtigen Flügellaschen 22 angebracht sind, können durch Klebstoff an der Außenlage 12 befestigt werden, um die Windel 10 um den Körper des Benutzers aufgerichtet anzuordnen.

Nachfolgend werden Verfahren zum Ausbilden der Öffnung 16 in der zweiten Decklage 14 und zum Versehen dieser Öffnung 16 mit ersten und zweiten elastischen Elementen 18, 19 unter Bezug auf Fig. 3 und 4 erläutert.

Wie in Fig. 3 gezeigt, werden zwei endlose elastische Elemente 19 zueinander beabstandet parallel und zwei weitere elastische Elemente 18 jeweils nach außen zu den jeweiligen elastischen Elementen 19 beabstandet ebenfalls parallel auf der Rückseite der zweiten Decklage 14 entlang ihrem Mittelbereich angeordnet, wobei sie um einen vorgegebenen Dehnungsprozentsatz gedehnt sind, und gleichzeitig auf dieser unter Verwendung von Heißschmelzkleber aufgeklebt. Wie aus Fig. 3 ersichtlich ist, ist die zweite Decklage 14 mit einer in Längsrichtung verlaufenden geraden Schnittlinie 24a versehen, die sich zwischen den beiden elastischen Elementen 19 im wesentlichen über eine Länge einer jeden einzelnen Windel erstreckt, und mit kreisbogenförmigen Schnittlinien 24b, die sich quer zur Schnittlinie 24a an den in Längsrichtung gegenüberliegenden Enden derselben zu den jeweiligen elastischen Elementen 18 erstrecken, um so ein Paar Klappen 17 zu bilden. Die entlang der Schnittlinie 24a verlaufenden Ränder werden umgeschlagen, um die jeweiligen elastischen Elemente 19 abzudecken und die jeweiligen Klappen 17 werden nach außen entlang den jeweiligen elastischen Elementen 18 gefaltet, um so die Öffnung 16 zu bilden. Während die Klappen 17 in einem in Längsrichtung gedehnten Zustand gehalten werden, werden die in Längsrichtung gegenüberliegenden Enden der Klappen 17 mittels Heißschmelzkleber mit der zweiten Decklage 14 verklebt. Die Längenabschnitte der elastischen Elemente 18, 19, die in Längsrichtung über die jeweiligen Schnittlinien 24a überstehen, können frei von Kleberauftrag sein und mittels Saugmitteln beispielsweise entfernt werden, nachdem die in Längsrichtung gegenüberliegenden Enden der Klappen 17 mit der zweiten Decklage verklebt wurden.

Während die Klappen 17 vorzugsweise aus der zweiten Decklage wie bei der gezeigten Ausführungsform gebildet werden, ist es auch möglich, diese Klappen 17 getrennt von der zweiten Decklage auszubilden und sie anschließend mit den in Querrichtung gegenüberliegenden Seitenrändern der Öffnung 16 zu verbinden.

Bezugszeichen L in Fig. 3 und 4 bezeichnet imaginäre Linien, entlang denen die zweite Decklage 14 geschnitten wird, um die einzelnen Windeln zu erhalten. Dieser Schneidvorgang wird durch Schneiden des endlosen Schichtkörpers in die einzelnen Windeln nach dem Aufbau des endlosen Schichtkörpers bewirkt.

Während die Ausführungsform als sogenannte Windel des offenen Typs dargestellt und beschrieben wurde, bei der die Klebebefestigungen zum Schließen der Hüftlinie um den Körper des Benutzers verwendet werden, ist die Erfindung ebenfalls auf sogenannte Windeln des Höschentyps (einschließlich Erziehungshöschen) anwendbar, die eine endlose Hüftlinie aufweisen.

Mit dem Anlegen der Windel 10 an dem Körper des Benutzers hebt sich, wie aus Fig. 1 und 2 ersichtlich ist, die Öffnung 16 aus der ersten Decklage 11 unter der Kontraktion der elastischen Elemente 18 heraus und die Klappen 17 werden entlang ihren inneren Seitenrändern, die die elastischen Elemente 18 enthalten, unter der Kontraktion der elastischen Elemente 19 nach oben und außen aufgerichtet, so daß diese Klappen 17 einander gegenüberliegen, um so die trichterartigen geneigten Seitenwände zu bilden. Ausscheidungen werden von diesen geneigten Seitenwänden der Öffnung 16 zugeführt. Flüssige Ausscheidungen werden durch die erste Decklage 11 in den Kern 13 absorbiert, während feste Ausscheidungen in eine zwischen der ersten Decklage 11 und der zweiten Decklage 14 gebildeten Tasche eingetragen werden.

Während es bevorzugt ist, die elastischen Elemente 18 entlang den Seitenrändern der Öffnung 16 vorzusehen, sind diese elastischen Elemente 18 nicht wesentlich für die Erfindung, da sie keinen bedeutenden Beitrag zur Bildung der geneigten Seitenwände durch die Klappen 17 leisten.

Die erfindungsgemäße Windel erlaubt das zuverlässige Zuführen von Ausscheidungen zur Öffnung, die in der zweiten Decklage ausgebildet ist, ohne die Befürchtung, daß Ausscheidungen teilweise über die Fläche der zweiten Decklage, die sich außerhalb der Öffnung erstreckt, verteilt werden könnten, was das größte Problem gewesen ist, das durch Windeln nach dem Stand der Technik nicht gelöst war, und erlaubt es zusätzlich, die Größe der Öffnung zu reduzieren.

## Patentansprüche

1. Wegwerfwindel, umfassend einen integralen Schichtkörper, der sich aus einer flüssigkeitsdurchlässigen ersten Decklage, einer flüssigkeitsundurchlässigen Außenlage, einem flüssigkeitsabsorbierenden Kern, der zwischen die erste Decklage und die Außenlage gelegt ist, und einer flüssigkeitsbeständigen zweiten Decklage zusammensetzt, die über die erste Decklage gelegt ist, wobei die zweite Decklage im wesentlichen in ihrem Mittelbereich eine Öffnung aufweist, die sich in Längsrichtung der zweiten Decklage weiter als in Querrichtung derselben erstreckt, wobei die zweite Decklage entlang ihrem äußeren Umfang mit der ersten Decklage verbunden ist und wobei in Längsrichtung elastische Elemente entlang dem Umfangsrand der Öffnung an der zweiten Decklage angebracht sind, wobei zwei flüssigkeitsbeständige Klappen von den in Querrichtung gegenüberliegenden Seitenrändern der Öffnung ausgehen, die wenigstens entlang ihren äußeren Seitenrändern jeweils mit elastischen Elementen versehen sind und die in Längsrichtung gegenüberliegenden Enden der jeweiligen Klappen nach außen gefaltet mit der zweiten Decklage verbunden sind und trichterartig geneigte Seitenwände bilden.

2. Wegwerfwindel nach Anspruch 1,
wobei die zweite Decklage in ihrem mittleren Bereich mit einer in Längsrichtung verlaufenden Schnittlinie versehen ist, die sich über eine gewünschte Länge erstreckt, und die zweite Decklage weiter an den in Längsrichtung gegenüberliegenden Enden der in Längsrichtung verlaufenden Schnittlinie mit quer verlaufenden Schnittlinien jeweils versehen ist, so daß Abschnitte der zweiten Decklage, die von diesen Schnittlinien begrenzt werden, nach außen gefaltet werden können, um die Klappen zu bilden.

3. Wegwerfwindeln nach Anspruch 1,
wobei zwischen der Unterfläche der Klappen und der Oberfläche der zweiten Decklage ein weiteres Paar elastischer Elemente vorgesehen ist, das sich entlang den jeweiligen Faltlinien der Klappen erstreckt.

4. Verfahren zum Herstellen einer Wegwerfwindel, umfassend einen integralen Schichtkörper, der sich aus einer flüssigkeitsdurchlässigen ersten Decklage, einer flüssigkeitsundurchlässigen Außenlage, einem flüssigkeitsabsorbierenden Kern, der zwischen die erste Decklage und die Außenlage gelegt ist, und einer flüssigkeitsbeständigen zweiten Decklage zusammensetzt, die über die erste Decklage gelegt ist, wobei die zweite Decklage im wesentlichen in ihrem Mittelbereich eine Öffnung aufweist, die sich in Längsrichtung der zweiten Decklage weiter als in Querrichtung derselben erstreckt, wobei die zweite Decklage entlang ihrem äußeren Umfang mit der ersten Decklage verbunden ist und wobei in Längsrichtung elastische Elemente entlang dem Umfangsrand der Öffnung an der zweiten Decklage angebracht sind, gekennzeichnet durch die folgenden Verfahrensschritte:
- Anordnen von zwei weiteren elastischen Elementen parallel und im Abstand voneinander auf der Rückseite der zweiten Decklage beidseits des Mittelbereichs in gedehntem Zustand,
- Aufkleben der elastischen Elemente in gedehntem Zustand mittels eines Klebers,
- Anbringen eines Längsschnitts in der Decklage in der Längsmittellinie entlang eines vorbestimmten Bereichs,
- Anlegen eines Schnitts quer zur Längsrichtung am Ort des jeweiligen Endes des Längsschnitts,
- Falten der so entstandenen Klappen nach außen,
- Dehnen der Klappen in Längsrichtung, und
- Ankleben der sich in Längsrichtung gegenüberliegenden umgefalteten Klappenenden im gedehnten Zustand auf der zweiten Decklage.

## Claims

1. A disposable happy, comprising an integral layered body, which is composed of a first top sheet permeable to liquids, an outer sheet impermeable to liquids, a liquid absorbing core which is disposed between the first top sheet and the outer sheet, a second top sheet resistant to liquids, the second top sheet being disposed over the first top sheet and having an opening substantially in its centre region which extends in the longitudinal direction of the second top sheet further than in the transverse direction thereof, wherein the second top sheet is joined to the first top sheet along its outer periphery and wherein elastic elements are fitted in the longitudinal direction along the peripheral edge of the opening in the second top sheet, wherein two flaps resistant to liquids extend from the side edges of the opening opposite one another in the transverse direction, being provided with the respective elastic elements at least along their outer side edges and respective ends of the flaps opposite one another in the longitudinal direction being connected to the second top sheet, folded out, and forming funnel-like inclined sidewalls.

2. A disposable nappy according to claim 1, wherein the second top sheet is provided in its central region with a cut line running in the longitudinal direction and which extends over a desired length, and the second top sheet is provided with respective transversely extending cut lines at the ends opposite one another in the longitudinal direction of the cut line extending in the longitudinal direction, so that sections of the second top sheet which are bounded by these cut lines can be folded out, in order to form the flaps.

3. A disposable nappy according to claim 1, wherein a further pair of elastic elements is provided between the lower surface of the flaps and the surface of the second top sheet, extending along the respective fold lines of the flaps.

4. A method of mixing a disposable nappy, comprising an integral layered body, which is composed of a first top sheet permeable to liquids, an outer sheet impermeable to liquids, a liquid absorbing core which is disposed between the first top sheet and the outer sheet, and second top sheet resistant to liquids, the second top sheet being disposed over the first top sheet and having an opening substantially in its centre region which extends in the longitudinal direction of the second top sheet further than in the transverse direction thereof, wherein the second top sheet is joined to the first top sheet along its outer periphery and wherein elastic elements are fitted in the longitudinal direction along the peripheral edge of the opening in the second top sheet, characterized by the following method steps:
- arranging two further elastic elements in the stretched state parallel to and spaced from one another on the rear side of the second top sheet on the two sides of the central region.
- bonding the elastic elements in the stretched state by means of an adhesive,
- forming a longitudinal slot in the top sheet in the longitudinal central line along a predetermined region,
- forming 8 cut transverse to the longitudinal direction at the place of the respective ends of the longitudinal cut,
- folding out the flaps thus formed,
- stretching the flaps in the longitudinal direction, and
- bonding the folded out flap ends opposite one another in the longitudinal direction, in the stretched state, on to the second top sheet.

## Revendications

1. Lange jetable comprenant un lamifié intégral composé d'une première couche de couverture perméable aux liquides, d'une couche extérieure imperméable aux liquides, d'un corps central absorbant les liquides emprisonné entre la première couche de couverture et la couche extérieure, et d'une seconde couche de couverture résistant aux liquides, posée par-dessus la première couche de couverture, la seconde couche de couverture présentant, pour l'essentiel dans sa partie médiane, une ouverture de dimension plus importante dans le sens longitudinal que dans le sens transversal de la seconde couche de courverture, la seconde couche de couverture étant jointe a la première couche de couverture le long de son pourtour extérieur, et des éléments élastiques étant appliqués longitudinalement sur la seconde couche de couverture, le long du bord périphérique de l'ouverture, lange dans lequel deux rabats résistant aux liquides se détachent des bords latéraux de l'ouverture, opposés en sens transversal, lesquels rabats sont respectivement pourvus d'éléments élastiques, au moins le long de leurs bords latéraux extérieurs, et les extrémités des rabats opposées dans le sens de la longueur, pliées vers l'extérieur, sont raccordées à la seconde couche de couverture et forment des pans latéraux ayant une pente pareille à celle d'un entonnoir.

2. Lange jetable selon la revendication 1, dans lequel la seconde couche de couverture est pourvue, dans sa partie médiane, d'une ligne de coupe longitudinale, qui s'étend sur une longueur voulue, et dans lequel, aux extrémités opposées en sens longitudinal de la ligne de coupe orientée longitudinalement, la seconde couche de couverture est encore pourvue, de chaque côté, de lignes de coupe à orientation transversale, de sorte que des portions de cette seconde couche de couverture, délimitées par ces lignes de coupe, peuvent être repliées vers l'extérieur, pour former les rabats.

3. Lange jetable selon la revendication 1, dans lequel une autre paire d'éléments élastiques, qui longent les lignes de pliage respectives des rabats, est prévue entre la surface inférieure des rabats et la surface supérieure de la seconde couche de couverture.

4. Procédé pour fabriquer un lange jetable comprenant un lamifié intégral composé d'une première couche de couverture perméable aux liquides, d'une couche extérieure imperméable aux liquides, d'un corps central absorbant les liquides emprisonné entre la première couche de couverture et la couche extérieure, et d'une seconde couche de couverture résistant aux liquides, posée par-dessus la première couche de couverture, la seconde couche de couverture présentant, pour l'essentiel dans sa partie médiane, une ouverture plus large dans le sens longitudinal que dans le sens transversal de la seconde couche de couverture, la seconde couche de couverture étant jointe à la première le long de son pourtour extérieur, et des éléments élastiques étant appliqués longitudinalement sur la seconde couche de couverture, le long du bord périphérique de l'ouverture, caractérisé par les opérations suivantes :
- application d'au moins deux éléments élastiques, parallèlement et à distance l'un de l'autre, au dos de la seconde couche de couverture, de part et d'autre de la partie médiane, à l'état étiré,
- collage des éléments élastiques à l'état étiré, au moyen d'un adhésif,
- formation d'une fente longitudinale dans la couche de couverture, sur la ligne médiane longitudinale, le long d'une zone définie au préalable,
- formation d'une fente transversalement à la longueur, à l'emplacement de chaque extrémité de la fente longitudinale,
- pliage, vers l'extérieur, des rabats ainsi formés,
- étirage des rabats dans le sens de la longueur, et
- collage, à l'état étiré, des extrémités des rabats opposées dans le sens longitudinal repliées, sur la seconde couche de couverture.
